# EUROPEAN PATENT APPLICATION

(11) **EP 4 844 403 A1**
(43) Date of publication of application: **25.11.2026**
(21) Application number: 26175083.0
(22) Date of filing: 28.04.2026
(51) Int. Cl.: G01N 33/49, G01N 33/50, G01N 33/72, G01N 33/569, G01N 21/82

(54) **BLOOD MEASURING DEVICE AND BLOOD MEASURING METHOD**

(30) Priority: 20.05.2025 JP 2025083772
(71) Applicant: Nihon Kohden Corporation, Shinjuku-ku Tokyo (JP)
(72) Inventor: Hamada, Tatsuya, Tokorozawa-shi, Saitama (JP); Kin, Daiken, Tokorozawa-shi, Saitama (JP); Saigusa, Yumi, Tokorozawa-shi, Saitama (JP)
(74) Representative: Grünecker Patent- und Rechtsanwälte PartG mbB

(57) **Abstract**

Provided is a blood measuring device that can perform D-dimer measurement and CBC measurement simultaneously using the same blood sample. The blood measuring device includes a CBC measurement unit configured to measure a CBC in whole blood containing EDTA which is an anticoagulant; a D-dimer measurement unit configured to measure a D-dimer value in the same whole blood as the whole blood used by the CBC measurement unit; and a correction unit configured to perform RBC correction, RGB correction, MCHC correction, or correction using a hemoglobin-related value on the D-dimer value measured by the D-dimer measurement unit.

## Description

### TECHNICAL FIELD

The invention relates to a blood measuring device and a blood measuring method.

### BACKGROUND ART

Patent Literature 1 (JP11-101798A) discloses that a latex immunoturbidimetric assay such as a CRP measurement is simultaneously performed as a CBC measurement in combination with a hemolytic reagent using whole blood containing an ethylenediaminetetraacetic acid (EDTA) salt such as K2-EDTA, 3K-EDTA, and Na2-EDTA2Na. According to such a device, serum and plasma conversion may be performed by volume correction using an HCT value.

On the other hand, it is desirable to perform a D-dimer measurement and the CBC measurement simultaneously using the same whole blood from a viewpoint of shortening measurement time. D-dimer is a diagnostic marker for a blood coagulation assay that indicates presence of a thrombus in a body by measuring cross-linked fibrin degradation products by plasmin. D-dimer has high diagnostic sensitivity but low disease specificity in thrombosis (in particular, deep venous thrombosis), and is used for exclusion diagnosis of thrombosis in combination with another test in addition to blood measurement items such as CBC and CRP.

### SUMMARY OF INVENTION

### TECHNICAL PROBLEM

D-dimer is a diagnostic marker for the blood coagulation assay, and is measured by a method such as latex agglutination immunoassay method, ELISA, chemiluminescence, and immunochromatography using a blood sample collected using a blood collection tube containing an anticoagulant such as sodium citrate and EDTA. However, D-dimer is contained in plasma, and is thus not measured in whole blood except by methods that involve B/F separation such as removing particles by centrifugation, filtration, and washing.

Further, in a D-dimer measurement, it is confirmed that a hemolysis state of a sample blood may negatively affect a measurement result due to an influence of particles, and a D-dimer value in a hemolyzed sample requires careful interpretation in diagnosis. In this way, it is difficult in the related art to perform the D-dimer measurement and the CBC measurement simultaneously using the same whole blood.

The invention is made to solve the above problem, and an object thereof is to provide a blood measuring device and a blood measuring method capable of simultaneously performing a D-dimer measurement and CBC measurement using the same whole blood.

### SOLUTION TO PROBLEM

The invention is achieved by following configurations.
(1)
   A blood measuring device includes: a CBC measurement unit configured to measure a CBC value in whole blood containing EDTA which is an anticoagulant; a D-dimer measurement unit configured to measure a D-dimer value in a same whole blood as the whole blood used by the CBC measurement unit; and a correction unit configured to correct the D-dimer value measured by the D-dimer measurement unit based on RBC correction, HGB correction, MCHC correction, or a hemoglobin-related value.
(2)
   A blood measuring method includes: a CBC measurement step of measuring a CBC value in whole blood containing EDTA which is an anticoagulant; a D-dimer measurement step of measuring a D-dimer value in a same whole blood as the whole blood used in the CBC measurement step; and a correction step of performing RBC correction, HGB correction, MCHC correction, or correction using a hemoglobin-related value on the D-dimer value measured in the D-dimer measurement step.

### EFFECTS OF INVENTION

According to the blood measuring device and the blood measuring method, a D-dimer value and a CBC value can be measured simultaneously using the same whole blood. Further, by hemolyzing the whole blood, it is possible to provide a measurement technique that does not require prior removal of blood cell by centrifugation or the like.

### BRIEF DESCRIPTION OF DRAWINGS

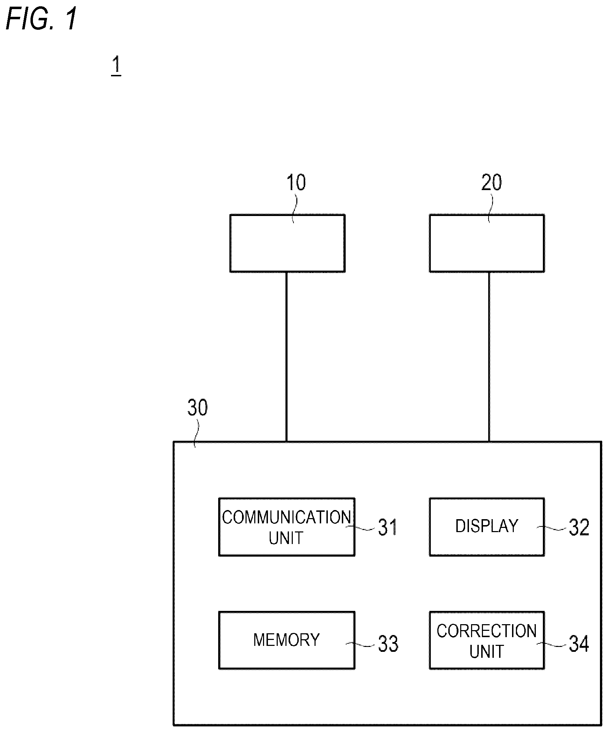

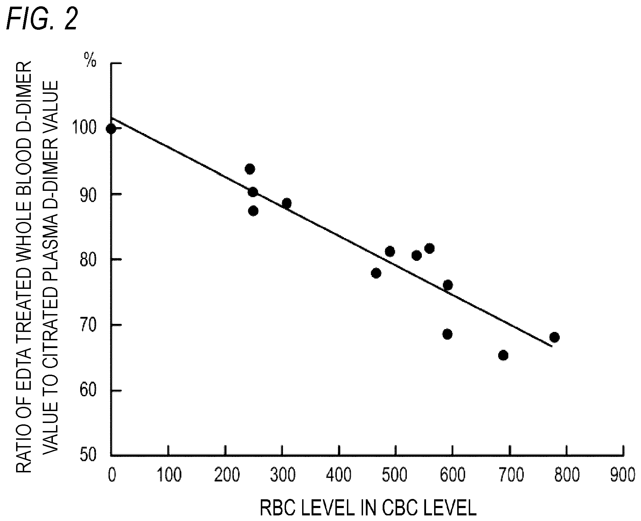

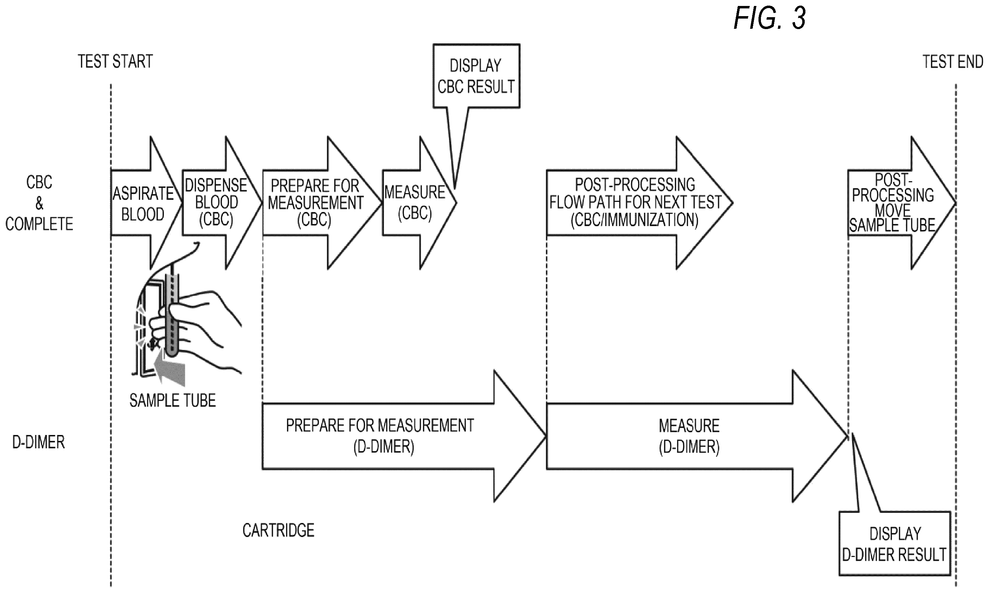

FIG. 1 is a block diagram illustrating a blood measuring device according to an embodiment of the invention. FIG. 2 is a graph for examining a correction formula. FIG. 3 illustrates a blood measuring method according to the present embodiment.

### DESCRIPTION OF EMBODIMENTS

Hereinafter, an embodiment of the invention will be described with reference to the drawings. In description of the drawings, the same elements are denoted by the same reference numerals, and redundant description thereof will be omitted. In the drawings, dimensional ratios are exaggerated for convenience of the description, and may be different from actual ratios.

Hereinafter, with reference to FIG. 1, a configuration of a blood measuring device 1 according to the present embodiment will be described. FIG. 1 is a block diagram illustrating the blood measuring device 1 according to the embodiment of the invention.

As illustrated in FIG. 1, the blood measuring device 1 according to the present embodiment can include a CBC measurement unit 10 that measures a CBC value, a D-dimer measurement unit 20 that measures a D-dimer value, and a controller 30 that controls various configurations. The CBC measurement unit 10 and the D-dimer measurement unit 20 are electrically connected to the controller 30.

The CBC measurement unit 10 measures a WBC (white blood cell) count, an RBC (red blood cell) count, a PLT (platelet) count, MCV (mean corpuscular volume), and HCT (hematocrit) by an electrical resistance method. The CBC measurement unit 10 further measures HGB (hemoglobin) by a cyanmethemoglobin method as a kind of a colorimetric method.

The D-dimer measurement unit 20 measures the D-dimer value by latex agglutination immunoassay method. The D-dimer measurement unit 20 can include a light source that emits measurement light for measuring the D-dimer value, and a photodetector that detects the measurement light that has passed through the D-dimer to be measured. The D-dimer measurement unit 20 optically measures the D-dimer value contained in whole blood to be measured. The D-dimer measurement unit 20 may include a plurality of light sources having different wavelengths, and may optically measure a hemoglobin-related value in addition to the D-dimer value. The controller 30 obtains an electric signal proportional to an amount of light detected by the photodetector.

The controller 30 can include a central processing unit (CPU), a read only memory (ROM), a random-access memory (RAM), and the like, and is implemented by various processors. The controller 30 starts a prescribed processing program and integrally controls units constituting the blood measuring device 1 to execute prescribed processing. As illustrated in FIG. 1, the controller 30 can include a communication unit 31, a display 32, a memory 33, and a correction unit 34.

The communication unit 31 is a network interface that controls wired or wireless communication (including short-range wireless communication) between the blood measuring device 1 and another device (communication destination such as an external server of a medical facility and various mobile terminals owned by a doctor in charge) connected via a communication network. The communication unit 31 transmits the measured CBC value in whole blood and the corrected D-dimer value in the whole blood to the communication destination.

The display 32 is implemented by a display device such as a liquid crystal display (LCD), an organic light emitting diode (OLED), and a micro light emitting diode (micro LED) display, and is an interface that outputs display content in a format (letters, numbers, figures, and the like) visible by a user. The display 32 displays, for example, the measured CBC value in the whole blood and the corrected D-dimer value in the whole blood, and also displays notification content (guidance display, display of measurement start or measurement end, and the like) necessary for operation.

The memory 33 comprises a known storage medium that stores various data used in the blood measuring device 1 such as various control programs necessary for driving the blood measuring device 1 and measurement data including values measured by the CBC measurement unit 10 and the D-dimer measurement unit 20.

The correction unit 34 performs RBC correction on the D-dimer value measured by the D-dimer measurement unit 20. More specifically, the correction unit 34 corrects the D-dimer value by performing mathematical correction to remove an interfering factor of the D-dimer value caused by an RBC that is generated when blood is hemolyzed.

Hereinafter, a purpose of performing the mathematical correction will be described. In a measurement of the D-dimer value, it is generally known that a measurement result deviates from a normal numerical value depending on a hemolysis state of sample blood. The present inventors have confirmed that there is a certain degree of reaction inhibition correlated with RBC, HGB, MCHC, hemoglobin-related value, and the like in the measurement of D-dimer in whole blood, and have found that the reaction inhibition is derived from blood cell components. By using a constant coefficient A based on the number of erythrocytes or the like, which will be described later, in accordance with a hemolytic reagent used in combination, it is possible to appropriately correct the D-dimer measurement by latex agglutination immunoassay method using whole blood containing EDTA.

Hereinafter, a method of correcting the D-dimer value using the RBC value will be described. The corrected D-dimer value is obtained by a multivariate polynomial of degree one or higher using the D-dimer value (measured value) and the RBC value.

With reference to FIG. 2, the polynomial using the D-dimer value and the RBC value will be described. In FIG. 2, a horizontal axis of the graph represents the RBC value as obtained in the CBC measurement, and a vertical axis represents the ratio of the D-dimer measured value by whole blood in EDTA-blood collection tube to the D-dimer value measured by plasma in a sodium citrate blood collection tube. As can be seen from the graph of FIG. 2, as the RBC value increases, the whole blood measured value tends to decrease. To return a numerical value of the vertical axis to 100%, a multivariate polynomial of degree one or higher using D-dimer and RBC is used.

Next, with reference to FIG. 3, a blood measuring method using the blood measuring device 1 will be described. FIG. 3 illustrates a blood measuring method according to the present embodiment.

Blood is aspirated from a blood collection tube containing EDTA and dispensed into a CBC measurement chamber and a D-dimer measurement cartridge. Then, a hemolytic reagent is added to the blood. The cartridge may contain a hemolytic reagent from a beginning.

Next, the CBC value in the blood dispensed into the CBC measurement chamber is measured by the CBC measurement unit 10. The measured CBC value is displayed on the display 32.

In addition, the D-dimer value in the blood dispensed into the D-dimer measurement cartridge is measured by the D-dimer measurement unit 20. Then, the D-dimer value measured by the D-dimer measurement unit is corrected by the correction unit 34, and the corrected D-dimer value is displayed on the display 32. The correction unit 34 may perform volume correction of EDTA or volume correction of HCT before performing the above-described correction. The D-dimer measurement may be performed in a chamber instead of a cartridge.

Here, the volume correction of EDTA is to correct an influence of blood dilution caused by a difference in a state of an anticoagulant between a sodium citrate blood collection tube and an EDTA salt-containing blood collection tube. The blood collected by the citrate blood collection tube is in a diluted state since sodium citrate is liquid. On the other hand, since the EDTA salt is solid, the blood collected in the EDTA salt-containing blood collection tube is not diluted, and a difference in dilution factor of blood occurs due to a difference in a type of a blood collection tube, and so that a difference in D-dimer value occurs. In the D-dimer measurement, a numerical value using blood containing sodium citrate is generally used as a true numerical value, and thus when a EDTA salt-containing blood collection tube is used, correction is performed using a dilution factor or the like.

In the volume correction of HCT, a numerical value using plasma as a specimen is generally used as a true numerical value, and thus correction is performed in a case of measurement using whole blood.

Then, a flow path is cleaned for a next measurement as post-processing.

As described above, the blood measuring device 1 according to the present embodiment can include the CBC measurement unit 10 configured to measure a CBC value in whole blood containing EDTA which is an anticoagulant; the D-dimer measurement unit 20 configured to measure a D-dimer value in a same whole blood as the whole blood used in the measurement of the CBC measurement unit 10; and the correction unit 34 configured to perform RBC correction on the D-dimer value measured by the D-dimer measurement unit 20. According to the blood measuring device 1 configured as described above, D-dimer measurement and a CBC measurement can be performed simultaneously using the same blood sample. Further, by hemolyzing the whole blood, a measurement technique can be provided that does not require prior removal of blood cell components (e.g., by centrifugation)..

Further, the correction unit 34 obtains the corrected D-dimer value by performing mathematical correction to remove an interfering factor of the D-dimer value caused by an RBC that is generated when the whole blood is hemolyzed. According to the blood measuring device 1 configured as described above, it is possible to appropriately perform D-dimer measurement and CBC measurement simultaneously using the same blood sample.

As described above, the blood measuring method according to the present embodiment can include a CBC measurement step of measuring a CBC value in whole blood containing EDTA which is an anticoagulant; a D-dimer measurement step of measuring a D-dimer value in a same whole blood used in the CBC measurement step; and a correction step of performing RBC correction on the D-dimer value measured in the D-dimer measurement step. According to the blood measuring method, it is possible to perform D-dimer measurement and CBC measurement simultaneously using the same blood sample.

Further, in the correction step, the D-dimer value is corrected by performing mathematical correction to remove an interfering factor of the D-dimer value caused by an RBC that is generated when the whole blood is hemolyzed. According to the blood measuring method, it is possible to appropriately perform D-dimer measurement and a CBC measurement simultaneously using the same blood sample.

The invention is not limited to the embodiment described above, and various modifications can be made within the scope of the claims.

For example, in the embodiment described above, the correction unit 34 corrects the D-dimer value by performing RBC correction. Alternatively, the D-dimer value may be corrected by performing HGB correction, MCHC correction, or correction using the hemoglobin-related value. When the RBC correction is performed and the D-dimer value cannot be accurately corrected, the HGB correction or the MCHC correction may be further performed.

## Claims

1. A blood measuring device comprising:
a CBC measurement unit configured to measure a CBC value in whole blood containing EDTA which is an anticoagulant;
a D-dimer measurement unit configured to measure a D-dimer value in a same whole blood as the whole blood used by the CBC measurement unit; and
a correction unit configured to correct the D-dimer value measured by the D-dimer measurement unit based on RBC correction, HGB correction, MCHC correction, or a hemoglobin-related value .

2. The device according to claim 1, wherein the correction unit corrects the D-dimer value by performing mathematical correction to remove an interfering factor of the D-dimer value caused by an RBC that is generated when the whole blood is hemolyzed.

3. A blood measuring method comprising:
a CBC measurement step of measuring a CBC value in whole blood containing EDTA which is an anticoagulant;
a D-dimer measurement step of measuring a D-dimer value in a same whole blood as the whole blood used in the CBC measurement step; and
a correction step of performing RBC correction, HGB correction, MCHC correction, or correction using a hemoglobin-related value on the D-dimer value measured in the D-dimer measurement step.

4. The method according to claim 3, wherein in the correction step, the D-dimer value is corrected by performing mathematical correction to remove an interfering factor of D-dimer caused by an RBC that is generated when the whole blood is hemolyzed.
